# EUROPEAN PATENT SPECIFICATION

(11) **EP 3 806 784 B1**
(45) Date of publication and mention of the grant of the patent: **02.10.2024**
(21) Application number: 18737772.6
(22) Date of filing: 14.06.2018
(51) Int. Cl.: A61F 2/95, A61F 2/24

(54) **SINGLE FIBER CONSTRAINING FOR IMPLANTABLE MEDICAL DEVICES**
EINZELFASERBEFESTIGUNG FÜR IMPLANTIERBARE MEDIZINPRODUKTE
ÉLÉMENT DE CONTRAINTE À UNE SEULE FIBRE POUR DISPOSITIFS MÉDICAUX IMPLANTABLES

(43) Date of publication of application: 21.04.2021
(73) Proprietor: W. L. Gore & Associates, Inc., Newark, DE 19711 (US)
(72) Inventor: SKELTON, Tyson J., Newark, Delaware 19711 (US)
(74) Representative: HGF
(86) International application number: PCT/US2018/037586
(87) International publication number: WO 2019/240800

(56) References cited:
- EP-A1- 0 878 175
- EP-A1- 0 878 175
- WO-A1-92/09245
- CA-A1- 2 456 046
- CN-A- 106 580 514
- CN-A- 106 580 514

## Description

### FIELD

The present disclosure relates to apparatuses, systems, and methods that include coverings for delivery of implantable medical devices. More specifically, the present disclosure relates to apparatuses, systems, and methods that include coverings for constraining an expandable device during device delivery.

### BACKGROUND

Stents and stent-grafts may be utilized to radially support a variety of tubular passages in the body, including arteries, veins, airways, gastrointestinal tracts, and biliary tracts. The preferred method of placing these devices has been to use specialized delivery systems to precisely place and deploy a device at the site to be treated. These delivery systems allow the practitioner to minimize the trauma and technical difficulties associated with device placements. Attributes of delivery systems include: low profile; ability to pass through introducer sheaths; ability to negotiate tortuous vasculature, smoothly and atraumatically; protection of constrained devices; and ability to accurately position and deploy the device.

Stents or stent-grafts may be deployed and plastically deform by using an inflatable balloon (e.g., balloon expandable stents) or to self-expand and elastically recover (e.g., "self expandable" stents) from a collapsed or constrained delivery diameter to an expanded and deployed diameter. Some stents are designed to elastically recover by being manufactured at their functional diameter of a material that has elastic recovery properties, and then radially compressed to be mounted on a delivery catheter.

These stent and stent-graft devices may be held, compressed, or constrained in the delivery configuration prior to and during delivery to a target location. The devices may be held in this compressed state for a prolonged period of time (e.g., after manufacture and prior to use). Different mechanisms or devices may be used to hold the stent and stent-graft devices in a delivery state and be removed to allow expansion of the stent and stent-graft devices at the target location. EP0878175 is directed to a procedure for inserting an implant comprising a tubular body with the aid of a flexible sleeve. The tube is implanted in a compressed state, held by a filament in a series of loops which are released after withdrawing the sleeve by pulling on the filament end situated outside the patient's body. The implanted tube is made, for example, from polyethylene terephthalate, polyester of PTFE, with a wall thickness of 0.10 mm and 0.8 mm. It can be between 6 and 16 cm long by 5 mm to 45 mm in diameter.
CN106580514 is directed to an implantation device of a duodenum stent characterized in that the device comprises a core tube which is used for bearing the duodenum stent; the wall of the core tube is provided with a first binding wire channel and a first stent binding wire window; and the first binding wire channel is internally provided with a first binding wire which is used for fixedly binding the duodenum stent. The implantation device of the duodenum stent can conveniently and reliably realize withdrawing after implantation of the duodenum stent. The main chamber of the core tube is provided with radial liquid discharging holes so that distilled water is injected through a handle liquid injection hole in retreating the implantation device and flows to a clearance between a stent sleeve and the implantation device. In injection of the distilled water, the implantation device of the duodenum stent is withdrawn and simultaneously rotated in a left-and-right direction for preventing packaging of the implantation device by the sleeve of the stent and furthermore preventing withdrawing of the implantation device from the stent sleeve.

### SUMMARY

The invention is defined by the appended claims.

A first aspect of the invention relates to an apparatus as defined in independent claim

In a second aspect there is provided a delivery system according to the invention, the delivery system including an implantable medical device; and a constraining mechanism as described above.

According to preferred embodiments each of the plurality of knots are in contact with adjacent ones of the plurality of knots when the constraining mechanism is in the constrained configuration.

According to preferred embodiments, the single fiber is configured to sequentially untie the plurality of knots in response to applied tension and release the constraining mechanism to allow expansion of the implantable medical device to a deployed configuration.

According to preferred embodiments, the implantable medical device includes a stent having a plurality of apices, and the single fiber is configured to release in sequence and avoid catching on the apices during release.

According to preferred embodiments, the plurality of knots are configured to maintain position relative to the implantable medical device in the constrained configuration prior to being released in sequence.

According to preferred embodiments, the plurality of knots are configured to lessen ramping of the implantable medical device prior to being released in sequence.

According to preferred embodiments, the plurality of knots are longitudinally aligned a longitudinal axis of the constraining mechanism.

According to preferred embodiments, the plurality of knots alternate sides of a longitudinal axis of the constraining mechanism.

According to preferred embodiments, the single fiber forms multiple loops are angled relative to the longitudinal axis of the constraining mechanism.

According to preferred embodiments, the multiple loops are substantially perpendicular to a longitudinal axis of the constraining mechanism formed by the plurality of knots.

According to preferred embodiments, the multiple loops are packed at a density with each loop being in physical contact with adjacent ones of the multiple loops.

For example, the multiple loops may be packed at a density configured to substantially gaplessly cover the implantable medical device and the density is between approximately 0.152 mm (0.006 inches) and 2.54 mm (0.1 inches).

According to preferred embodiments, the implantable medical device comprises a drug eluting coating, and the multiple loops of the constraining mechanism are configured to lessen release of the drug eluting coating prior to the constraining mechanism releasing to allow expansion of the implantable medical device to a deployed configuration.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is a top plan view of a catheter with a constraining mechanism, according to some embodiments.
FIG. 2 is an illustration of an example constraining mechanism, according to some embodiments.
FIG. 3 is an illustration of an example constraining mechanism and implantable medical device, according to some embodiments.
FIG. 4 is an illustration of another example constraining mechanism and implantable medical device, according to some embodiments.

The accompanying drawings are included to provide a further understanding of the disclosure and are incorporated in and constitute a part of this specification, illustrate embodiments, and together with the description serve to explain the principles of the disclosure.

### DETAILED DESCRIPTION

Persons skilled in the art will readily appreciate that various aspects of the present disclosure can be realized by any number of methods and apparatus configured to perform the intended functions as long as they fall under the scope of the claims. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward apparatuses, methods, and systems that include a constraining mechanism configured to hold, compress, or constrain an implantable medical device (e.g., a stent or stent-graft) in a delivery configuration prior to and during delivery to a target location. The constraining mechanism includes a single fiber. The single fiber, as compared to certain sheaths, sleeves or multiple fiber constraining mechanisms, may constrain an implantable medical device at a smaller profile.

The single fiber, wraps the device circumferentially with each circumferential wrap of the single fiber being secured with a loop. The loop may include a loop knitting pattern along the length of the device with a plurality of knots. In addition, the single fiber constraining mechanism may facilitate deployment of the implantable medical device by avoiding catching on the implantable medical device and avoiding undesired pre-deployment of the device as discussed in further detail below. In particular, compared multiple fiber constraining mechanisms, the single fiber constraining mechanism can lessen the opportunity for catching and pre-deployment on the device.

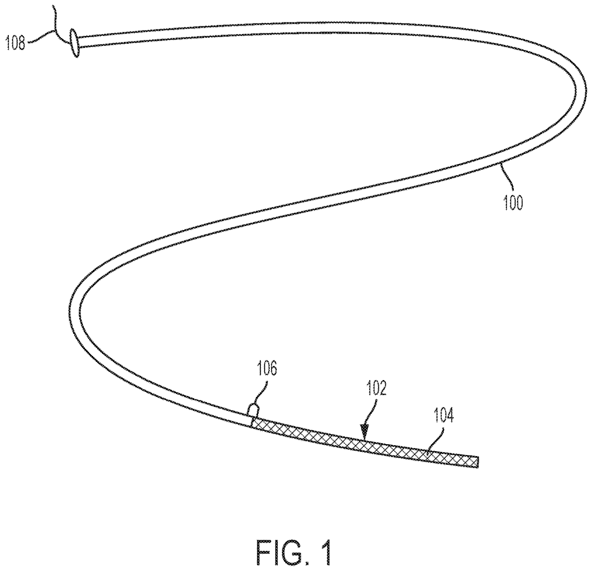

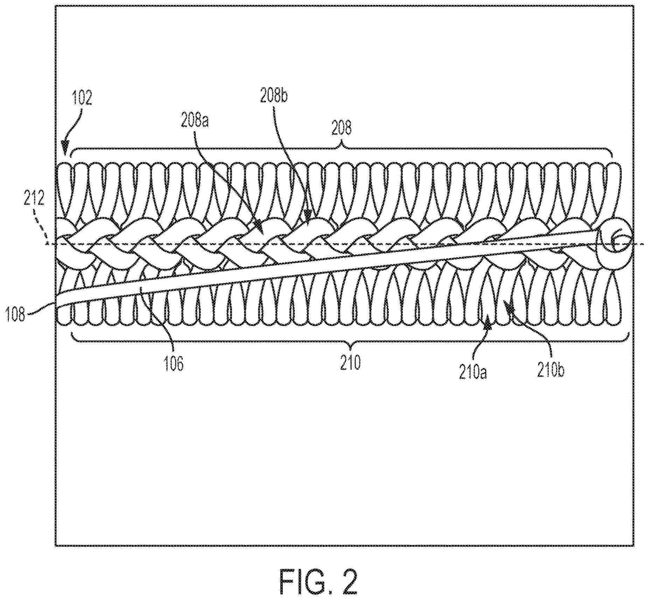

FIG. 1 is a top plan view of a catheter 100 with a constraining mechanism 102, according to some embodiments. As shown in FIG. 1, the constraining mechanism 102 is configured to constrain an implantable medical device 104 to a delivery configuration. The constraining mechanism 102 includes a single fiber 106 arranged about the implantable medical device 104 having a plurality of knots to maintain the constraining mechanism 102 in a constrained configuration. The single fiber 106 of the constraining mechanism 102, as shown in further detail with reference to FIGs. 2-4, includes a series of knots.

The constraining mechanism 102 is arranged along a length of the implantable medical device 104. The constraining mechanism 102 is also circumferentially arranged about the implantable medical device 104 and may substantially cover the implantable medical device 104 for delivery. In addition, and as shown in FIG. 1, the single fiber 106 includes a series of knots and is also circumferentially arranged about the implantable medical device 104 over the length of the implantable medical device 104 with the single fiber 106 being not knotted or otherwise wrapped proximal to the implantable medical device 104. The single fiber 106 may be arranged within a lumen (not shown) of the catheter 100 and extend toward a proximal end of the catheter 100 that is arranged external to a patient during delivery of the implantable medical device 104. The single fiber 106 includes a proximal end 108 that a user may apply tension to in order to release the constraining mechanism 102 and deploy the implantable medical device 104.

In certain instances, the single fiber 106 releases similar to a rip cord such that the knots sequentially release along the length of the implantable medical device 104. As is explained in greater detail below, the constraining mechanism 102 is formed by knitting together the single fiber 106 directly on the implantable medical device 104. As contrasted to prior multiple fiber constraining mechanisms which are knotted together and then subsequently arranged about a constrained device, the constraining mechanism 102 is formed directly on the implantable medical device 104 according to various examples. The implantable medical device 104 may be a stent, stent-graft, a balloon, or a similar device.

FIG. 2 is an illustration of an example constraining mechanism 102, according to some embodiments. The constraining mechanism 102 may be included a portion of a delivery system (*e.g.,* a catheter and implantable medical device as shown in FIG. 1). FIG. 2 shows the constraining mechanism 102 in a constrained configuration in which an implantable medical device (not shown) is held to a diameter less than a deployed, expanded or working diameter. The constraining mechanism 102 is configured to constrain an implantable medical device to a delivery configuration. In addition, the constraining mechanism 102 includes a single fiber 106 arranged having a plurality of knots 208 to maintain the constraining mechanism 102 in a constrained configuration. The plurality of knots 208 are configured and arranged such that at least two of the plurality of knots 208 are in contact in the constrained configuration as shown in FIG. 2.

As highlighted in FIG. 2, adjacent knots 208a-b of the plurality of knots 208 are in physical contact. The knots 208a-b being in contact increases the density of the constraining mechanism 102 as compared to prior multi-fiber constrain devices. In certain instances, each of the plurality of knots 208 are in contact with adjacent knots as shown in FIG. 2. In addition, and in certain instances, the plurality of knots 208 are longitudinally aligned along longitudinal axis 212 of the constraining mechanism 102. The alignment of the plurality of knots 208 can facilitate densely packing of the plurality of knots 208. In certain instances, the alignment of the plurality of knots 208 and at least two adjacent knots 208a-b of the plurality of knots 208 being in physical contact relates to an amount of force applied by the constraining mechanism 102. In certain instances, the fiber 106 may be wrapped around the implantable medical device at tension in a range between 300 g and over 1 kg.

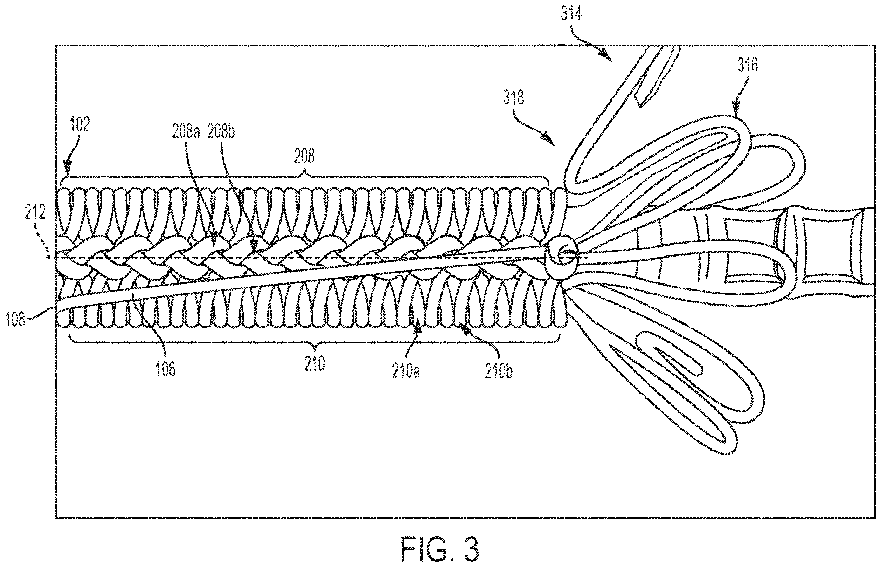

In addition, and as discussed in further detail with reference to FIG. 3, the alignment of the plurality of knots 208 and at least two adjacent knots 208a-b of the plurality of knots 208 being in physical contact facilitates deployment of an implantable medical device by avoiding catching on the implantable medical device and avoiding undesired pre-deployment of the device as discussed in further detail below.

In certain instances, the single fiber 106 forms multiple loops 210 arranged circumferentially about the implantable medical device. In addition, and as shown in FIG. 2, the multiple loops 210 are packed at a density such that at least two loops 210a-b of the multiple loops 210 are in physical contact. The loops 210 are unknotted portions of the single fiber 102 between knots of the plurality of knots 208. The multiple loops 210 may be arranged circumferentially about an implantable medical device and in certain instances, the multiple loops 210 are substantially perpendicular to the longitudinal axis 212 of the constraining mechanism 102.

As compared to prior multi-fiber constraining devices, the single fiber 106 is configured to prevent non-sequential tensioning and un-tensioning (e.g., releasing) that can complicate deployment. The plurality of knots 208 of the single fiber 106 forming the constraining mechanism 102 are an interlocking structure that unravels as a coherent interwoven rip cord by unknotting the plurality of knots 208 on the single fiber 106. As tension is applied to the proximal end 108 of the single fiber 106, the plurality of knots 208 release in sequence. This process will continue along the entire length of the device until each of the plurality of knots 208 disengage as one long, continuous, un-knotted single fiber 106.

FIG. 3 is an illustration of an example constraining mechanism 102 and implantable medical device 314, according to some embodiments. The constraining mechanism 102 is formed of a single fiber 106 that includes a plurality of knots 208 arranged along a longitudinal axis 212 of the constraining mechanism 102. In addition, the single fiber 106 forms multiple loops 210 arranged perpendicular to the longitudinal axis 212 of the constraining mechanism. The multiple loops 210 are also arranged circumferentially about the implantable medical device 314. The single fiber 106 includes a proximal end 108 that a user may apply tension to in order to release the constraining mechanism 102 and deploy the implantable medical device 104.

According to the invention, at least two adjacent knots 208a-b of the plurality of knots 208 are in physical contact and at least two loops 210a-b of the multiple loops 210 are in physical contact. The single fiber 106 may be configured to sequentially untie the plurality of knots 208 in response to applied tension and release the constraining mechanism 102 to allow expansion of the implantable medical device 314 to a deployed configuration.

In FIG. 3, the implantable medical device 314 is shown in a partially deployed configuration with the constraining mechanism 102 having been partially released. The implantable medical device 314 may be a stent that includes multiple apices 316 with a single apex highlighted in FIG. 3 for ease of illustration. As noted above, the single fiber 106 facilitates deployment by avoiding catching on the implantable medical device 314. Releasing the plurality of knots 208 in sequence avoid being caught on the apices 316 during release. The plurality of knots 208 may be released along the longitudinal axis 212 to avoid snagging on the apices 316. The single fiber 106 avoids shifting axially relative to the implantable medical device 314. In addition, releasing the plurality of knots 208 in sequence maintains a consistent deployment force during deployment of the expandable medical 314, which may avoid misdeployment or shifting of the constraining mechanism 102.

In certain instances, the plurality of knots 208 are configured to maintain position relative to the implantable medical device 314 in the constrained configuration prior to being released in sequence. The single fiber 106 may be configured to lessen ramping of the implantable medical device 314 prior to the plurality of knots 208 being released in sequence. As shown in FIG. 3, the expandable medical 314 begins to expand to a larger diameter after release of the constraining mechanism 102. The expandable medical 314 may be have an angle 318 between the portions held by the constraining mechanism 102 and portions that have been expanded or are beginning to expand. Due to the angle 318 and the expandable device 314 expending a force to deploy to the deployed diameter, prior devices may shift due to ramping of the implantable medical device 314. The axial shifting of the constraining mechanism 102 could result in pre-deployment or the constraint to get caught on apices of the implantable medical device 314. The single fiber 106, however, is able to lessen ramping of the implantable medical device 314 by maintaining a location of each of the plurality of knots 208, relative to the implantable medical device 314, as the plurality of knots 208 are released in sequence. The single fiber 106, in this manner, lessens undesired or pre-deployment of the implantable medical device 314.

The multiple loops 210 of the constraining mechanism 102 may be packed at a density such that each loop 210 is in physical contact with adjacent ones 21 0a-c of the multiple loops 210 as noted above. In certain instances, the multiple loops 210 are packed at a density configured to substantially gaplessly cover the implantable medical device 314. In certain instances, the density is between approximately 0.152 mm (0.006 inches) and 2.5 mm (0.1 inches). Further, the implantable medical device 314 may include a drug eluting coating and the multiple loops 210 of the constraining mechanism 102 are configured to lessen release of the drug eluting coating prior to the constraining mechanism 102 releasing to allow expansion of the implantable medical device 314 to a deployed configuration.

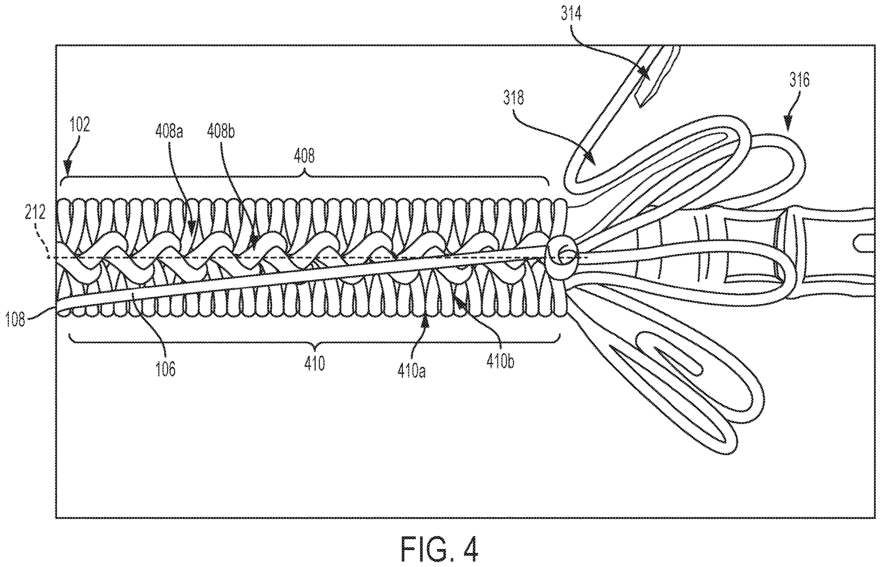

FIG. 4 is an illustration of another example constraining mechanism 102 and implantable medical device 314, according to some embodiments. As discussed in further detail above with reference to FIGs. 2-3, the constraining mechanism 102 is formed of a single fiber 106 that includes a plurality of knots 408. In addition, the single fiber 106 forms multiple loops 410 arranged about a circumference of the constraining mechanism 102 between the plurality of knots 408.

As shown in FIG. 4, the plurality of knots 408 are arranged on alternating sides of a longitudinal axis 212 of the constraining mechanism 102. In addition, the multiple loops 410 are also arranged circumferentially about the implantable medical device 314. The single fiber 106 forms the multiple loops 410 in an angled configuration circumferentially about the implantable medical device 314. The multiple loops 410 may be angled relative to the longitudinal axis 21 of the constraining mechanism 102. According to the invention, at least two adjacent knots 408a-b of the plurality of knots 408 are in physical contact. Further, at least two loops 410a-b of the multiple loops 410 are in physical contact. The single fiber 106 may be configured to sequentially untie the plurality of knots 408 in response to applied tension and release the constraining mechanism 102 to allow expansion of the implantable medical device 314 to a deployed configuration.

In FIG. 4, the implantable medical device 314 is shown in a partially deployed configuration with the constraining mechanism 102 being partially released. In certain instances, the plurality of knots 408 are configured to maintain position relative to the implantable medical device 314 in the constrained configuration prior to being released in sequence. As shown in FIG. 4, the implantable medical device 314 begins to expand to a larger diameter after release of the constraining mechanism 102. The implantable medical device 314 may be have an angle 318 between the portions held by the constraining mechanism 102 and portions that have been expanded or are beginning to expand. Due to the angle 318 and the expandable device 314 exerts a force to deploy to the deployed diameter, prior devices may shift due to ramping of the implantable medical device 314. The single fiber 106, however, lessens ramping of the implantable medical device 314 by maintaining a location of each of the plurality of knots 408, relative to the implantable medical device 314, as the plurality of knots 408 are released in sequence. The single fiber 106, in this manner, lessens undesired or pre-deployment of the implantable medical device 314.

The device shown in FIG. 4 is provided as an example of the various features of the constraining mechanism 102 and, although the combination of those illustrated features is clearly within the scope of invention, that example and its illustration is not meant to suggest the inventive concepts provided herein are limited from fewer features, additional features, or alternative features to one or more of those features shown in FIG. 4. For example, in various embodiments, the constraining mechanism 102 shown in FIG. 4 may include the density of loops described with reference to FIG. 3. It should also be understood that the reverse is true as well. One or more of the components depicted in FIG. 4 can be employed in addition to, or as an alternative to components depicted in FIGs. 2-3. For example, the alternating knots 408 of the constraining mechanism 102 shown in FIG. 4 may be employed in connection with the constraining mechanism 102 of FIG. 2-3.

The materials used to make the single fiber 106 of the present invention are likewise open to modification and customization for given applications. For most uses discussed herein the single fiber 106 used to form the constraining mechanism 102 may include: polytetrafluoroethylene (PTFE); expanded PTFE; silk; thermoplastic threads such as polypropylene; polyamide (nylon); various plastic or metal materials (e.g., stainless steel or nickel-titanium (nitinol) alloy); and bioresorbable materials, such as PLA or PGA. Particularly preferred for use in covering implantable medical devices are polytetrafluoroethylene (PTFE) threads, and especially expanded PTFE threads, such as threads available from W. L. Gore & Associates, Inc., Elkton, Md., under the trademark RASTEX^{®} or sutures available from W. L. Gore & Associates, Inc., Flagstaff, Ariz., under the trademark GORE-TEX^{®}.

## Claims

1. An apparatus comprising:
a constraining mechanism (102) configured to constrain an implantable
medical device (104, 314) to a delivery configuration, the constraining mechanism (102) including:
a single fiber (106) arranged about the implantable medical device (104, 304) having a plurality of knots (208) configured to be released in sequence and the single fiber (106) forming multiple loops (210) arranged circumferentially about the implantable medical device (104, 314) in a constrained configuration, the multiple loops (210) being packed at a density such that at least two loops (210a-b) of the multiple loops (210) are in physical contact in the constrained configuration, wherein at least two of the plurality of knots (208) are in contact in the constrained configuration, and
the single fiber (106) having a substantially unknotted structure in a non-constrained configuration.

2. A delivery system (100) comprising:
an implantable medical device (104, 314); and
the constraining mechanism (102) of claim 1.

3. The system (100) of claim 2, wherein each of the plurality of knots (208) are in contact with adjacent ones of the plurality of knots (208) when the constraining mechanism (102) is in the constrained configuration.

4. The system (100) of any one of claims 2-3, wherein the single fiber (106) is configured to sequentially untie the plurality of knots (208) in response to applied tension and release the constraining mechanism (102) to allow expansion of the implantable medical device (104, 314) to a deployed configuration.

5. The system (100) of claim 4,
wherein the implantable medical device (104, 314) includes a stent having a plurality of apices (316), and the single fiber (106) is configured to release in sequence and avoid catching on the apices (306) during release.

6. The system (100) of claim 4 or 5, wherein the plurality of knots (208) are configured to maintain position relative to the implantable medical device (104, 314) in the constrained configuration prior to being released in sequence.

7. The system of claim 6, wherein the plurality of knots (208) are configured to lessen ramping of the implantable medical device (104) prior to being released in sequence.

8. The system (100) of any one of claims 2-7, wherein the plurality of knots (208) are longitudinally aligned a longitudinal axis (212) of the constraining mechanism (102).

9. The system (100) of any one of claims 2-7, wherein the plurality of knots alternate sides of a longitudinal axis (212) of the constraining mechanism (102).

10. The system (100) of claim 9, wherein the multiple loops (210) are angled relative to the longitudinal axis (212) of the constraining mechanism (102).

11. The system (100) of any one of claims 2 - 10, wherein the multiple loops (210) are substantially perpendicular to a longitudinal axis (212) of the constraining mechanism (102) formed by the plurality of knots (208).

12. The system (200) of claim 11, wherein the multiple loops (210) are packed at a density with each loop (210) being in physical contact with adjacent ones (210a-c) of the multiple loops (210).

13. The system (100) of any one of claims 10- 12, wherein the implantable medical device (314) comprises a drug eluting coating, and the multiple loops (210) of the constraining mechanism (102) are configured to lessen release of the drug eluting coating prior to the constraining mechanism (102) releasing to allow expansion of the implantable medical device (314) to a deployed configuration.

## Patentansprüche

1. Vorrichtung, umfassend:
einen Befestigungsmechanismus (102), der dazu konfiguriert ist, ein implantierbares Medizinprodukt (104, 314) in einer Abgabekonfiguration zu befestigen, wobei der Befestigungsmechanismus (102) Folgendes enthält:
eine Einzelfaser (106), die um das implantierbare Medizinprodukt (104, 314) angeordnet ist und eine Vielzahl von Knoten (208) aufweist, die dazu konfiguriert sind, nacheinander freigegeben zu werden, und wobei die Einzelfaser (106) vielfache Schlaufen (210) bildet, die in einer befestigten Konfiguration in Umfangsrichtung um das implantierbare Medizinprodukt (104, 314) angeordnet sind, wobei die vielfachen Schlaufen (210) mit einer solchen Dichte gepackt sind, dass mindestens zwei Schlaufen (210a-b) der vielfachen Schlaufen (210) in der befestigten Konfiguration in physischem Kontakt sind, wobei mindestens zwei der Vielzahl von Knoten (208) in der befestigten Konfiguration in Kontakt sind, und
wobei die Einzelfaser (106) eine im Wesentlichen unverknotete Struktur in einer nicht befestigten Konfiguration aufweist.

2. Abgabesystem (100), umfassend:
ein implantierbares Medizinprodukt (104, 314); und
den Befestigungsmechanismus (102) nach Anspruch 1.

3. System (100) nach Anspruch 2, wobei jeder der Vielzahl von Knoten (208) mit benachbarten Knoten der Vielzahl von Knoten (208) in Kontakt ist, wenn sich der Befestigungsmechanismus (102) in der befestigten Konfiguration befindet.

4. System (100) nach einem der Ansprüche 2-3, wobei die Einzelfaser (106) dazu konfiguriert ist, die Vielzahl von Knoten (208) als Reaktion auf einen aufgebrachten Zug nacheinander zu lösen und den Befestigungsmechanismus (102) freizugeben, um eine Ausdehnung des implantierbaren Medizinprodukts (104, 314) in eine entfaltete Konfiguration zu ermöglichen.

5. System (100) nach Anspruch 4,
wobei das implantierbare Medizinprodukt (104, 314) einen Stent enthält, der eine Vielzahl von Spitzen (316) aufweist, und die Einzelfaser (106) dazu konfiguriert ist, nacheinander freizugeben und während der Freigabe ein Hängenbleiben an den Spitzen (316) zu verhindern.

6. System (100) nach Anspruch 4 oder 5, wobei die Vielzahl von Knoten (208) dazu konfiguriert ist, ihre Position relativ zu dem implantierbaren Medizinprodukt (104, 314) in der befestigten Konfiguration beizubehalten, bevor sie nacheinander freigegeben werden.

7. System nach Anspruch 6, wobei die Vielzahl von Knoten (208) dazu konfiguriert ist, ein Aufweiten des implantierbaren Medizinprodukts (104) zu verringern, bevor sie nacheinander freigegeben werden.

8. System (100) nach einem der Ansprüche 2-7, wobei die Vielzahl von Knoten (208) in Längsrichtung auf eine Längsachse (212) des Befestigungsmechanismus (102) ausgerichtet ist.

9. System (100) nach einem der Ansprüche 2-7, wobei sich die Vielzahl von Knoten abwechselnd auf den Seiten einer Längsachse (212) des Befestigungsmechanismus (102) befindet.

10. System (100) nach Anspruch 9, wobei die vielfachen Schlaufen (210) relativ zu der Längsachse (212) des Befestigungsmechanismus (102) abgewinkelt sind.

11. System (100) nach einem der Ansprüche 2-10, wobei die vielfachen Schlaufen (210) im Wesentlichen senkrecht zu einer Längsachse (212) des Befestigungsmechanismus (102), der durch die Vielzahl von Knoten (208) gebildet wird, sind.

12. System (200) nach Anspruch 11, wobei die vielfachen Schlaufen (210) mit einer solchen Dichte gepackt sind, dass jede Schlaufe (210) in physischem Kontakt mit benachbarten Schlaufen (210a-c) der vielfachen Schlaufen (210) ist.

13. System (100) nach einem der Ansprüche 10-12, wobei das implantierbare Medizinprodukt (314) eine Arzneimittel eluierende Beschichtung umfasst, und die vielfachen Schlaufen (210) des Befestigungsmechanismus (102) dazu konfiguriert sind, eine Freigabe der Arzneimittel eluierenden Beschichtung zu verringern, bevor der Befestigungsmechanismus (102) freigegeben wird, um eine Ausdehnung des implantierbaren Medizinproduktes (314) in eine entfaltete Konfiguration zu ermöglichen.

## Revendications

1. Appareil comprenant :
un mécanisme de contrainte (102) conçu pour contraindre un dispositif médical implantable (104, 314) dans une configuration de pose, le mécanisme de contrainte (102) comprenant :
une fibre unique (106) disposée autour du dispositif médical implantable (104, 314) comportant une pluralité de noeuds (208) conçus pour être libérés en séquence et la fibre unique (106) formant de multiples boucles (210) disposées circonférentiellement autour du dispositif médical implantable (104, 314) dans une configuration contrainte, les multiples boucles (210) étant conditionnées à une densité telle qu'au moins deux boucles (210a à b) des multiples boucles (210) soient en contact physique dans la configuration contrainte, au moins deux de la pluralité de noeuds (208) étant en contact dans la configuration contrainte, et
la fibre unique (106) comportant une structure sensiblement non nouée dans une configuration non contrainte.

2. Système de pose (100) comprenant :
un dispositif médical implantable (104, 314) ; et
le mécanisme de contrainte (102) selon la revendication 1.

3. Système (100) selon la revendication 2, chacun de la pluralité de noeuds (208) étant en contact avec des noeuds adjacents de la pluralité de noeuds (208) lorsque le mécanisme de contrainte (102) se trouve dans la configuration contrainte.

4. Système (100) selon l'une quelconque des revendications 2 à 3, ladite fibre unique (106) étant conçue pour dénouer séquentiellement la pluralité de noeuds (208) en réponse à une tension appliquée et libérer le mécanisme de contrainte (102) pour permettre l'expansion du dispositif médical implantable (104, 314) dans une configuration déployée.

5. Système (100) selon la revendication 4,
ledit dispositif médical implantable (104, 314) comprenant un stent comportant une pluralité de sommets (316), et ladite fibre unique (106) étant conçue pour se libérer en séquence et éviter de s'accrocher aux sommets (316) pendant la libération.

6. Système (100) selon la revendication 4 ou 5, ladite pluralité de noeuds (208) étant conçus pour maintenir leur position par rapport au dispositif médical implantable (104, 314) dans la configuration contrainte avant d'être libérés en séquence.

7. Système selon la revendication 6, ladite pluralité de noeuds (208) étant conçus pour réduire l'augmentation graduelle du dispositif médical implantable (104) avant d'être libérés en séquence.

8. Système (100) selon l'une quelconque des revendications 2 à 7, ladite pluralité de noeuds (208) étant alignés longitudinalement sur l'axe longitudinal (212) du mécanisme de contrainte (102).

9. Système (100) selon l'une quelconque des revendications 2 à 7, ladite pluralité de noeuds alternant sur les côtés de l'axe longitudinal (212) du mécanisme de contrainte (102).

10. Système (100) selon la revendication 9, lesdites multiples boucles (210) étant inclinées par rapport à l'axe longitudinal (212) du mécanisme de contrainte (102).

11. Système (100) selon l'une quelconque des revendications 2 à 10, lesdites multiples boucles (210) étant sensiblement perpendiculaires à l'axe longitudinal (212) du mécanisme de contrainte (102) formé par la pluralité de noeuds (208).

12. Système (200) selon la revendication 11, lesdites multiples boucles (210) étant conditionnées à une densité, chaque boucle (210) étant en contact physique avec les boucles adjacentes (210a à c) parmi les multiples boucles (210).

13. Système (100) selon l'une quelconque des revendications 10 à 12, ledit dispositif médical implantable (314) comprenant un revêtement à élution de médicament, et lesdites multiples boucles (210) du mécanisme de contrainte (102) étant conçues pour réduire la libération du revêtement à élution de médicament avant la libération du mécanisme de contrainte (102) de manière à permettre l'expansion du dispositif médical implantable (314) vers une configuration déployée.
